# EUROPEAN PATENT APPLICATION

(11) **EP 3 466 420 A1**
(43) Date of publication of application: **10.04.2019**
(21) Application number: 18206136.6
(22) Date of filing: 29.04.2010
(51) Int. Cl.: A61K 31/225, A61P 25/14, A61P 25/28

(54) **DIMETHYL FUMARATE FOR THE TREATMENT OF FRIEDREICH ATAXIA**

(30) Priority: 29.04.2009 US 17379709 P; 04.05.2009 US 17527009 P
(62) Divisional of application: 17202547.0
(71) Applicant: Biogen MA Inc., Cambridge, MA 02142 (US)
(72) Inventor: Lukashev, Matvey, Tewksbury, MA Massachusetts 02142 (US)
(74) Representative: Pohlman, Sandra M.

(57) **Abstract**

Methods of treating a subject having a condition characterized by at least one of neurodegeneration and neuroinflammation are provided. Methods of reducing astrogliosis in a subject having a condition characterized by increased astrogliosis are also provided. Methods of providing neuroprotection to a subject in need thereof are also provided.

## Description

This applications claims priority to U.S. Provisional Patent Application Nos. 61/173,797, filed April 29, 2009, and 61/175,270, filed May 4, 2009. The entire disclosures of both of those applications are hereby incorporated herein by reference.

Provided are methods of treating a subject having a condition characterized by at least one of neurodegeneration and neuroinflammation, by administering to the subject a therapeutically effective amount of at least one compound of Formula I, or a pharmaceutically acceptable salt thereof. Methods of reducing astrogliosis in a subject having a condition characterized by increased astrogliosis, the method comprising administering to the subject a therapeutically effective amount of at least one compound of Formula I or a pharmaceutically acceptable salt thereof are also provided. Methods of providing neuroprotection to a subject in need thereof, the method comprising administering to the subject a therapeutically effective amount of at least one compound of Formula I or a pharmaceutically acceptable salt thereof are also provided. Screening methods to identify a compound as a candidate agent to treat a condition characterized by at least one of neurodegeneration and neuroinflammation are also provided.

Astrocytes are the major cellular component of the brain. These glial cells account for about 90% of the overall brain mass and outnumber neurons five- to ten-fold in the human adult brain. In the early 1980s, two types of astrocytes were characterized: fibrous and protoplasmic. Fibrous astrocytes (type 1) have a star-like shape, are normally found in white matter, and have long processes that run between myelinated fibers, blood capillaries, and form vascular end-feet structures around the blood-brain barrier (BBB). Conversely, protoplasmic astrocytes (type 2) are ramified, have short processes, which envelop neuronal processes and inhabit the grey matter.

Activation of glial cells, microglia and astrocytes, has been implicated as a mechanism contributing to the pathobiology of neurodegenerative and neuroinflammatory diseases, including multiple sclerosis (MS). In early stages of disease, astrocytes can secrete cytokines and chemokines that recruit inflammatory cells. As the disease progresses, astrogliosis is thought to contribute to glial scarring, axonal damage, and demyelination. Microglia have been shown to have a critical role in the development and progression of EAE pathogenesis. Pro-inflammatory cytokines produced by microglia exacerbate disease and chemokines recruit leukocytes to sites of inflammation. Dimethyl fumarate (DMF) is the active component of the experimental therapeutic BG00012 currently in Phase III relapsing-remitting MS (RRMS) clinical trials. In the Phase IIb RRMS study, BG00012 significantly reduced gadolinium-enhancing brain lesions. In preclinical studies, DMF has been shown to inhibit CNS inflammation in murine and rat EAE. It has now been found that DMF can inhibit astrogliosis and microglial activations associated with EAE.

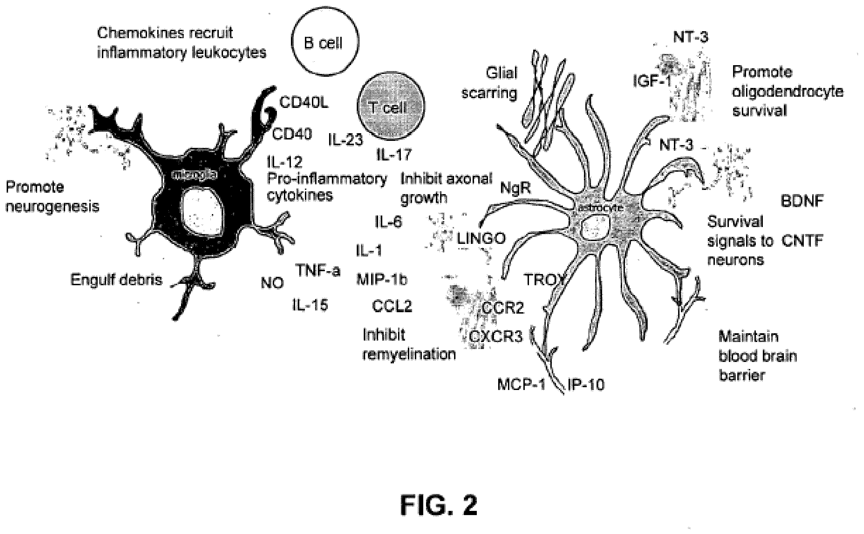

Certain non-limiting aspects of the role of microglia and astrocytes in neuroinflammatory pathogenesis are shown in Figure 2. Among the evidence for a role of astrogliosis in neurodegeneration and neuroinflammation is evidence from the study of Multiple Sclerosis (MS), one example of a disease characterized by neurodegeneration and neuroinflammation. In that model activation of astrocytes and microglia occurs prior to the onset of disease symptoms and axonal damage in rodent MS models. Additionally, selective ablation of microglia reduces EAE disease severity and inflammation. Clinical evidence from MS patients provides further evidence for a role of astrocites, because astrogliosis increases during disease flares. Additionally, activated astrocytes have been reported as a prominent cell type in secondary progressive MS, and re-activation of microglia is implicated as a driver of MS disease flares.

Multiple sclerosis (MS) is an autoimmune disease with the autoimmune activity directed against central nervous system (CNS) antigens. The disease is characterized by inflammation in parts of the CNS, leading to the loss of the myelin sheathing around neuronal axons (demyelination), axonal loss, and the eventual death of neurons, oligodenrocytes and glial cells. For a comprehensive review of MS and current therapies, see, e.g., McAlpine's Multiple Sclerosis, by Alastair Compston et al., 4th edition, Churchill Livingstone Elsevier, 2006.

An estimated 2,500,000 people in the world suffer from MS. It is one of the most common diseases of the CNS in young adults. MS is a chronic, progressing, disabling disease, which generally strikes its victims some time after adolescence, with diagnosis generally made between 20 and 40 years of age, although onset may occur earlier. The disease is not directly hereditary, although genetic susceptibility plays a part in its development. MS is a complex disease with heterogeneous clinical, pathological and immunological phenotype.

There are four major clinical types of MS: 1) relapsing-remitting MS (RR-MS), characterized by clearly defined relapses with full recovery or with sequelae and residual deficit upon recovery; periods between disease relapses characterized by a lack of disease progression; 2) secondary progressive MS (SP-MS), characterized by initial relapsing remitting course followed by progression with or without occasional relapses, minor remissions, and plateaus; 3) primary progressive MS (PP-MS), characterized by disease progression from onset with occasional plateaus and temporary minor improvements allowed; and 4) progressive relapsing MS (PR-MS), characterized by progressive disease onset, with clear acute relapses, with or without full recovery; periods between relapses characterized by continuing progression.

Clinically, the illness most often presents as a relapsing-remitting disease and, to a lesser extent, as steady progression of neurological disability. Relapsing-remitting MS (RR-MS) presents in the form of recurrent attacks of focal or multifocal neurologic dysfunction. Attacks may occur, remit, and recur, seemingly randomly over many years. Remission is often incomplete and as one attack follows another, a stepwise downward progression ensues with increasing permanent neurological deficit. The usual course of RR-MS is characterized by repeated relapses associated, for the majority of patients, with the eventual onset of disease progression. The subsequent course of the disease is unpredictable, although most patients with a relapsing-remitting disease will eventually develop secondary progressive disease. In the relapsing-remitting phase, relapses alternate with periods of clinical inactivity and may or may not be marked by sequelae depending on the presence of neurological deficits between episodes. Periods between relapses during the relapsing-remitting phase are clinically stable. On the other hand, patients with progressive MS exhibit a steady increase in deficits, as defined above and either from onset or after a period of episodes, but this designation does not preclude the further occurrence of new relapses.

MS pathology is, in part, reflected by the formation of focal inflammatory demyelinating lesions in the white matter, which are the hallmarks in patients with acute and relapsing disease. In patients with progressive disease, the brain is affected in a more global sense, with diffuse but widespread (mainly axonal) damage in the normal appearing white matter and massive demyelination also in the grey matter, particularly, in the cortex.

Most current therapies for MS are aimed at the reduction of inflammation and suppression or modulation of the immune system. As of 2006, the available treatments for MS reduce inflammation and the number of new episodes but not all of the treatments have an effect on disease progression. A number of clinical trials have shown that the suppression of inflammation in chronic MS rarely significantly limits the accumulation of disability through sustained disease progression, suggesting that neuronal damage and inflammation are independent pathologies. Thus, in advanced stages of MS, neurodegeneration appears to progress even in the absence of significant inflammation. Therefore, slowing demyelination, or promoting CNS remyelination as a repair mechanism, or otherwise preventing axonal loss and neuronal death are some of the important goals for the treatment of MS, especially, in the case of progressive forms of MS such as SP-MS.

Fumaric acid esters, such as dimethyl fumarate (DMF), have been previously proposed for the treatment of MS (see, e.g., Schimrigk et al., Eur. J. Neurol., 2006, 13(6):604-10; Drugs R&D, 2005, 6(4):229-30; U.S. Patent No. 6,436,992).

Provided herein is evidence that dimethyl fumarate (DMF) reduces astrocyte activation in vivo and in vitro and that DMF inhibits inflammatory cytokines and pro-inflammatory signaling induced by lipopolysaccharide (LPS) stimulation of primary astrocytes.

Provided are methods of treating a subject having a condition characterized by at least one symptom chosen from neurodegeneration and neuroinflammation. In some embodiments the method includes administering to the subject a therapeutically effective amount of at least one compound of Formula I: 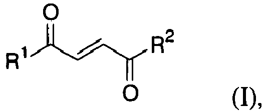 wherein R¹ and R² are independently selected from OH, O⁻, and (C₁₋₆)alkoxy, provided that at least one of R¹ and R² is (C₁₋₆)alkoxy, or a pharmaceutically acceptable salt thereof. In some embodiments, the administration to the subject of the therapeutically effective amount of the at least one compound of Formula I, or a pharmaceutically acceptable salt thereof, results in the supression of expression in the subject of at least one gene selected from Ccl20, Ccl3, Ccl4, Cxcll, Cxcl10, Cxcl2, Cxcl3, Cxcl6, IL1a, II1b, Tnf, Ifit3, Nfkbia, Nfkbiz, Tnfaip2, and Zc3h12a. In some embodiments, the condition characterized by at least one symptom chosen from neurodegeneration and neuroinflammation is further characterized by increased expression of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a. In some embodiments, the administration to the subject of the therapeutically effective amount of the at least one compound of Formula I results in upregulation of expression in the subject of at least one gene selected from Gsta2, Gsta3, Gclc, Ggt1, Txnrd1, Srxn1, Sqstm1, and Nqo1. In some embodiments increased expression of at least one gene selected from Gsta2, Gsta3, Gclc, Ggt1, Txnrd1, Srxn1, Sqstm1, and Nqo1 is achieved in the absence of supression of expression in the subject of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a. In some embodiments, the at least one compound is formulated as a pharmaceutical composition comprising the at least one compound and at least one pharmaceutically acceptable vehicle chosen from carriers, adjuvants, and excipients. In some embodiments, the at least one compound is chosen from dimethyl fumarate and monomethyl fumarate. In some embodiments, the only active agent administered to the subject is dimethyl fumarate (DMF). In some embodiments, the only active agent administered to the subject is monomethyl fumarate (MMF). In some embodiments, the only active agents administered to the subject are DMF and MMF. In some embodiments, the at least one compound is administered in an amount and for a period of time sufficient to reduce at least one of neurodegeneration and neuroinflammation in the subject. In some embodiments, the condition characterized by at least one symptom chosen from neurodegeneration and neuroinflammation is selected from Adrenal Leukodystrophy (ALD), Alcoholism, Alexander's disease, Alper's disease, Alzheimer's disease, Amyotrophic lateral sclerosis (Lou Gehrig's Disease), Ataxia telangiectasia, Batten disease (also known as Spielmeyer-Vogt-Sjögren-Batten disease), Bovine spongiform encephalopathy (BSE), Canavan disease, Cerebral palsy, Cockayne syndrome, Corticobasal degeneration, Creutzfeldt-Jakob disease, Familial Fatal Insomnia, Frontotemporal lobar degeneration, Huntington's disease, HIV-associated dementia, Kennedy's disease, Krabbe's disease, Lewy body dementia, Neuroborreliosis, Machado-Joseph disease (Spinocerebellar ataxia type 3), Multiple System Atrophy, Multiple sclerosis, Narcolepsy, Niemann Pick disease, Parkinson's disease, Pelizaeus-Merzbacher Disease, Pick's disease, Primary lateral sclerosis, Prion diseases, Progressive Supranuclear Palsy, Refsum's disease, Sandhoff disease, Schilder's disease, Subacute combined degeneration of spinal cord secondary to Pernicious Anaemia, Spielmeyer-Vogt-Sjogren-Batten disease (also known as Batten disease), Spinocerebellar ataxia, Spinal muscular atrophy, Steele-Richardson-Olszewski disease, Tabes dorsalis, Toxic encephalopathy, LHON (Leber's Hereditary optic neuropathy), MELAS (Mitochondrial Encephalomyopathy; Lactic Acidosis; Stroke), MERRF (Myoclonic Epilepsy; Ragged Red Fibers), PEO (Progressive External Opthalmoplegia), Leigh's Syndrome, MNGIE (Myopathy and external ophthalmoplegia; Neuropathy; Gastro-Intestinal; Encephalopathy), Kearns-Sayre Syndrome (KSS), NARP, Hereditary Spastic Paraparesis, Mitochondrial myopathy, and Friedreich Ataxia. In some embodiments, the condition characterized by at least one of neurodegeneration and neuroinflammation is Multiple Sclerosis (MS). In some embodiments, the subject does not have MS. In some embodiments the subject has relapsing remitting multiple sclerosis and treatment reduces the frequency of clinical relapses and delays the accumulation of physical disability. In some embodiments the subject has relapsing remitting multiple sclerosis and treatment reduces the frequency of clinical exacerbations.

Also provided are methods of reducing astrogliosis in a subject having a condition characterized by increased astrogliosis. In some embodiments the methods include administering to the subject a therapeutically effective amount of at least one compound of Formula I: 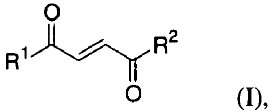 wherein R¹ and R² are independently selected from OH, O⁻, and (C₁₋₆)alkoxy, or a pharmaceutically acceptable salt thereof. In some embodiments, the administration to the subject of the therapeutically effective amount of the at least one compound of Formula I, or a pharmaceutically acceptable salt thereof, results in the supression of expression in the subject of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a. In some embodiments, the administration to the subject of the therapeutically effective amount of the at least one compound of Formula I results in upregulation of expression in the subject of at least one gene selected from Gsta2, Gsta3, Gclc, Ggt1, Txnrd1, Srxn1, Sqstm1, and Nqo1. In some embodiments increased expression of at least one gene selected from Gsta2, Gsta3, Gclc, Ggt1, Txnrd1, Srxn1, Sqstml, and Nqol is achieved in the absence of supression of expression in the subject of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a. In some embodiments, the at least one compound is formulated as a pharmaceutical composition comprising the at least one compound and at least one pharmaceutically acceptable vehicle chosen from carriers, adjuvants, and excipients. In some embodiments, the at least one compound is formulated as a pharmaceutical composition comprising the at least one compound and at least one pharmaceutically acceptable vehicle chosen from carriers, adjuvants, and excipients. In some embodiments, the at least one compound is chosen from dimethyl fumarate and monomethyl fumarate. In some embodiments, the only active agent administered to the subject is dimethyl fumarate (DMF). In some embodiments, the only active agent administered to the subject is monomethyl fumarate (MMF). In some embodiments, the only active agents administered to the subject are DMF and MMF. In some embodiments, the at least one compound is administered in an amount and for a period of time sufficient to reduce at least one of neurodegeneration and neuroinflammation in the subject. In some embodiments, the condition characterized by increased astrogliosis is selected from Adrenal Leukodystrophy (ALD), Alcoholism, Alexander's disease, Alper's disease, Alzheimer's disease, Amyotrophic lateral sclerosis (Lou Gehrig's Disease), Ataxia telangiectasia, Batten disease (also known as Spielmeyer-Vogt-Sjögren-Batten disease), Bovine spongiform encephalopathy (BSE), Canavan disease, Cerebral palsy, Cockayne syndrome, Corticobasal degeneration, Creutzfeldt-Jakob disease, Familial Fatal Insomnia, Frontotemporal lobar degeneration, Huntington's disease, HIV-associated dementia, Kennedy's disease, Krabbe's disease, Lewy body dementia, Neuroborreliosis, Machado-Joseph disease (Spinocerebellar ataxia type 3), Multiple System Atrophy, Multiple sclerosis, Narcolepsy, Niemann Pick disease, Parkinson's disease, Pelizaeus-Merzbacher Disease, Pick's disease, Primary lateral sclerosis, Prion diseases, Progressive Supranuclear Palsy, Refsum's disease, Sandhoff disease, Schilder's disease, Subacute combined degeneration of spinal cord secondary to Pernicious Anaemia, Spielmeyer-Vogt-Sjogren-Batten disease (also known as Batten disease), Spinocerebellar ataxia, Spinal muscular atrophy, Steele-Richardson-Olszewski disease, Tabes dorsalis, Toxic encephalopathy, LHON (Leber's Hereditary optic neuropathy), MELAS (Mitochondrial Encephalomyopathy; Lactic Acidosis; Stroke), MERRF (Myoclonic Epilepsy; Ragged Red Fibers), PEO (Progressive External Opthalmoplegia), Leigh's Syndrome, MNGIE (Myopathy and external ophthalmoplegia; Neuropathy; Gastro-Intestinal; Encephalopathy), Kearns-Sayre Syndrome (KSS), NARP, Hereditary Spastic Paraparesis, Mitochondrial myopathy, and Friedreich Ataxia. In some embodiments, the condition characterized by at least one of neurodegeneration and neuroinflammation is Multiple Sclerosis (MS). In some embodiments, the subject does not have MS. In some embodiments the subject has relapsing remitting multiple sclerosis and treatment reduces the frequency of clinical relapses and delays the accumulation of physical disability. In some embodiments the subject has relapsing remitting multiple sclerosis and treatment reduces the frequency of clinical exacerbations

Also provided are methods of identifying a compound as a candidate neuroprotection agent. In some embodiments the methods include a) inducing at least one of neurodegeneration and neuroinflammation in a target cell, tissue, or mammal, b) measuring expression of at least one marker of at least one of neurodegeneration and neuroinflammation in the target cell, tissue, or mammal in the presence of the compound, and c) measuring expression of at least one marker of at least one of neurodegeneration and neuroinflammation in the target cell, tissue, or mammal in the absence of the compound, wherein, if the expression of at least one marker of at least one of neurodegeneration and neuroinflammation is reduced in the presence of the compound relative to its expression in the absence of the compound, the compound is identified as a candidate neuroprotection agent. In some embodiments the methods further comprise d) measuring astrogliosis in the presence of the at least one compound, and e) measuring astrogliosis in the absence of the at least once compound, wherein, astrogliosis is reduced in the presence of the compound relative to the level of astrogliosis in the absence of the compound. In some embodiments the at least one marker is the expression level of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a.

Also provided are methods of providing neuroprotection to a subject in need thereof. In some embodiments the methods include administering to the subject a therapeutically effective amount of at least one compound of Formula I: 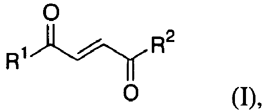 wherein R¹ and R² are independently selected from OH, O⁻, and (C₁₋₆)alkoxy, provided that at least one of R¹ and R² is (C₁₋₆)alkoxy, or a pharmaceutically acceptable salt thereof, wherein the administration to the subject of the therapeutically effective amount of the at least one compound of Formula I, or a pharmaceutically acceptable salt thereof, results in the supression of expression in the subject of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a. In some embodiments, the the condition characterized by at least one symptom chosen from neurodegeneration and neuroinflammation is further characterized by increased expression of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a. In some embodiments, the administration to the subject of the therapeutically effective amount of the at least one compound of Formula I results in upregulation of expression in the subject of at least one gene selected from Gsta2, Gsta3, Gclc, Ggt1, Txnrd1, Srxn1, Sqstm1, and Nqo1. In some embodiments increased expression of at least one gene selected from Gsta2, Gsta3, Gclc, Ggt1, Txnrd1, Srxn1, Sqstml, and Nqol is achieved in the absence of supression of expression in the subject of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a. In some embodiments, the at least one compound is formulated as a pharmaceutical composition comprising the at least one compound and at least one pharmaceutically acceptable vehicle chosen from carriers, adjuvants, and excipients. In some embodiments, the at least one compound is chosen from dimethyl fumarate and monomethyl fumarate. In some embodiments, the only active agent administered to the subject is dimethyl fumarate (DMF). In some embodiments, the only active agent administered to the subject is monomethyl fumarate (MMF). In some embodiments, the only active agents administered to the subject are DMF and MMF. In some embodiments, the at least one compound is administered in an amount and for a period of time sufficient to reduce at least one of neurodegeneration and neuroinflammation in the subject. In some embodiments, the condition characterized by at least one symptom chosen from neurodegeneration and neuroinflammation is selected from Adrenal Leukodystrophy (ALD), Alcoholism, Alexander's disease, Alper's disease, Alzheimer's disease, Amyotrophic lateral sclerosis (Lou Gehrig's Disease), Ataxia telangiectasia, Batten disease (also known as Spielmeyer-Vogt-Sjögren-Batten disease), Bovine spongiform encephalopathy (BSE), Canavan disease, Cerebral palsy, Cockayne syndrome, Corticobasal degeneration, Creutzfeldt-Jakob disease, Familial Fatal Insomnia, Frontotemporal lobar degeneration, Huntington's disease, HIV-associated dementia, Kennedy's disease, Krabbe's disease, Lewy body dementia, Neuroborreliosis, Machado-Joseph disease (Spinocerebellar ataxia type 3), Multiple System Atrophy, Multiple sclerosis, Narcolepsy, Niemann Pick disease, Parkinson's disease, Pelizaeus-Merzbacher Disease, Pick's disease, Primary lateral sclerosis, Prion diseases, Progressive Supranuclear Palsy, Refsum's disease, Sandhoff disease, Schilder's disease, Subacute combined degeneration of spinal cord secondary to Pernicious Anaemia, Spielmeyer-Vogt-Sjogren-Batten disease (also known as Batten disease), Spinocerebellar ataxia, Spinal muscular atrophy, Steele-Richardson-Olszewski disease, Tabes dorsalis, Toxic encephalopathy, LHON (Leber's Hereditary optic neuropathy), MELAS (Mitochondrial Encephalomyopathy; Lactic Acidosis; Stroke), MERRF (Myoclonic Epilepsy; Ragged Red Fibers), PEO (Progressive External Opthalmoplegia), Leigh's Syndrome, MNGIE (Myopathy and external ophthalmoplegia; Neuropathy; Gastro-Intestinal; Encephalopathy), Kearns-Sayre Syndrome (KSS), NARP, Hereditary Spastic Paraparesis, Mitochondrial myopathy, and Friedreich Ataxia. In some embodiments, the condition characterized by at least one of neurodegeneration and neuroinflammation is Multiple Sclerosis (MS). In some embodiments, the subject does not have MS. In some embodiments the subject has relapsing remitting multiple sclerosis and treatment reduces the frequency of clinical relapses and delays the accumulation of physical disability. In some embodiments the subject has relapsing remitting multiple sclerosis and treatment reduces the frequency of clinical exacerbations.

### BRIEF DESCRIPTION OF THE FIGURES

Figure 1A: DMF dose response in a rat EAE model.
Figure 1B: Glial cell inhibition by BG00012.
Figure 2: Various molecular mediators of the roles of astrocytes and microglia in neuroinflammatory pathogenesis.
Figure 3A: Astrocyte staining of rat spinal cords with and without DMF treatment.
Figure 3B: Morphometric quantitation using Aperio color deconvolution.
Figure 3C: Ventral grey matter and white matter regions.
Figure 3D: Morphometric quantitation of positive GFAP staining in ventral grey and white matter.
Figure 4A: BG200012 inhibits expression of GFAP.
Figure 4B: BG200012 inhibits LPS induced TNF expression.
Figure 4C: In vitro PD response to BG00012 in astrocytes.
Figure 4D: Astrocyte viability following BG00012 treatment.
Figure 5: BG00012 inhibits inflammatory cytokines and pro-inflammatory signaling induced by LPS stimulation of primary astrocytes.
Figure 6A: Glutathione levels in cultured astrocytes.
Figure 6B: Metabolic activity of cultured astrocytes.
Figure 6C: Cell viability of cultured astrocytes.
Figure 7A: Raw fluorescence traces from cells treated with MMF.
Figure 7B: Ca⁺⁺ mobilization in cells treated with MMF.
Figure 7C: Non-linear regression analysis of data.
Figure 8A: Cell viability of cultures astrocytes.
Figure 8B: Metabolic activity of cultured astrocytes.
Figure 8C: ATP levels in cultured astrocytes.
Figure 9: DMF and MMF increase cellular levels of Nrf2 in primary rat and human asrtrocytes.
Figure 10: Canonical signaling pathways stimulated by DMF in primary rat astrocytes.
Figure 11: Major cellular functions affected by DMF in primary ras astrocytes.
Figure 12: DMF treatment diminishes myelin loss during EAE (rat spinal cords).

A condition characterized by at least one of neurodegeneration and neuroinflammation is a condition in which either or both of those processes leads to a failure of the subjects nervous system to function normally. The loss of normal function may be located in either or both of the central nervous system (e.g., the brain, spinal cord) and the peripheral nervous system. Examples of such conditions include, but are not limed to, Adrenal Leukodystrophy (ALD), Alcoholism, Alexander's disease, Alper's disease, Alzheimer's disease, Amyotrophic lateral sclerosis (Lou Gehrig's Disease), Ataxia telangiectasia, Batten disease (also known as Spielmeyer-Vogt-Sjögren-Batten disease), Bovine spongiform encephalopathy (BSE), Canavan disease, Cerebral palsy, Cockayne syndrome, Corticobasal degeneration, Creutzfeldt-Jakob disease, Familial Fatal Insomnia, Frontotemporal lobar degeneration, Huntington's disease, HIV-associated dementia, Kennedy's disease, Krabbe's disease, Lewy body dementia, Neuroborreliosis, Machado-Joseph disease (Spinocerebellar ataxia type 3), Multiple System Atrophy, Multiple sclerosis, Narcolepsy, Niemann Pick disease, Parkinson's disease, Pelizaeus-Merzbacher Disease, Pick's disease, Primary lateral sclerosis, Prion diseases, Progressive Supranuclear Palsy, Refsum's disease, Sandhoff disease, Schilder's disease, Subacute combined degeneration of spinal cord secondary to Pernicious Anaemia, Spielmeyer-Vogt-Sjogren-Batten disease (also known as Batten disease), Spinocerebellar ataxia, Spinal muscular atrophy, Steele-Richardson-Olszewski disease, Tabes dorsalis, Toxic encephalopathy, LHON (Leber's Hereditary optic neuropathy), MELAS (Mitochondrial Encephalomyopathy; Lactic Acidosis; Stroke), MERRF (Myoclonic Epilepsy; Ragged Red Fibers), PEO (Progressive External Opthalmoplegia), Leigh's Syndrome, MNGIE (Myopathy and external ophthalmoplegia; Neuropathy; Gastro-Intestinal; Encephalopathy), Kearns-Sayre Syndrome (KSS), NARP, Hereditary Spastic Paraparesis, Mitochondrial myopathy, and Friedreich Ataxia.

In some embodiments, administration of at least one compound or pharmaceutically acceptable salt thereof, as described herein, to a patient gives rise to "neuroprotection," or said another way, the effect of administering the compound to the patient is neuroprotection. Neuroprotection comprises at least one of maintenance, salvage, recovery, and regeneration of the nervous system, its cells, structure, and function following injury or damage. In some embodiments neuroprotection comprises at least one of primary neuroprotection and secondary neuroprotection. "Primary neuroprotection" is protection comprising direct modulation of the structure and/or function of neural cells residing within the CNS (at least one cell type selected from neurons, oligodendrocytes, astrocytes, and microglia). "Secondary neuroprotection" is protection comprising modulation of the structure or function of at least one cell type that typically resides outside the CNS (*e.g*. immune cells). In secondary neuroprotection the at least one compound or pharmaceutically acceptable salt thereof acts directly or indirectly on the at least one cell type that typically resides outside the CNS to modulate the structure and/or function of that at least one cell type. That at least one cell type then modulates, directly or otherwise, the structure and/or function of neural cells residing within the CNS (at least one cell type selected from neurons, oligodendrocytes, astrocytes, and microglia). In some embodiments, neuroprotection comprises a lessening of the severity or rate of neurodegeneration or neuroinflammation in a subject. "Maintenance" of the nervous system, its cells, structure, and function comprises embodiments in which the at least one compound or pharmaceutically acceptable salt thereof is administered to a subject prior to development of at least one sign or symptom of a disease or condition disclosed herein and reduces the eventual severity of the disease or condition and/or reduces the rate of onset of the disease and/or condition.

In some embodiments the condition to be treated is characterized by increased expression of pro-inflammatory genes, such as in neural cells of the subject. In the case of a subject experiencing astrogliosis, for example, expression of at least one pro-inflammatory gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a is increased in the subject. In some embodiments, administration of at least one compound or pharmaceutically acceptable salt thereof, as described herein, to the subject, results in suppression of expression of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a.

Certain examples of neuroprotective genes are also disclosed herein, namely Gsta2, Gsta3, Gclc, Ggt1, Txnrd1, Srxn1, Sqstm1, and Nqo1. In some embodiments, administration of at least one compound or pharmaceutically acceptable salt thereof, as described herein, to the subject, results in upregulation of at least one gene selected from Gsta2, Gsta3, Gclc, Ggt1, Txnrd1, Srxn1, Sqstm1, and Nqo1.

The term "therapeutically effective amount" refers to that amount of a compound or pharmaceutically acceptable salt thereof which results in prevention or delay of onset or amelioration of at least one symptom of a condition characterized by neurodegeneration or neuroinflammation in a subject, or an attainment of a desired biological outcome, such as reduced astrogliosis.

In some embodiments the expression level of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, Zc3h12a, Gsta2, Gsta3, Gclc, Ggt1, Txnrd1, Srxn1, Sqstm1, and Nqo1 is measured in a subject. In some embodiments, expression of the gene is measured by determining the expression level of an mRNA for that gene. In some embodiments, expression of the gene is measured by determining the expression level of a protein product encoded by the gene. In some embodiments the protein product is measured in cerebrospinal fluid of the subject. In some embodiments expression level is measured at at least one time point selected from prior to initiation of treatment, during treatment, and after treatment.

The term "treating" refers to administering a therapy in an amount, manner, and/or mode effective to prevent or delay onset of or amelioration of at least one symptom of a condition characterized by neurodegeneration or neuroinflammation in a subject, to either a statistically significant degree or to a degree detectable to one skilled in the art. An effective amount, manner, or mode can vary depending on the subject and may be tailored to the subject. For neurological disorders referred herein, the treatment offered by the method of this invention aims at improving the conditions (or lessening the detrimental effects) of the disorders and not necessarily at completely eliminating or curing the disorders.

Unless otherwise specified, the term "MMF" refers to monomethyl fumarate in the form of acid (methyl hydrogen fumarate, also known as "MHF") as well as to its corresponding salts.

In some embodiments, the methods of the invention comprise administering to a subject having the condition a therapeutically effective amount of at least one compound of Formula I:

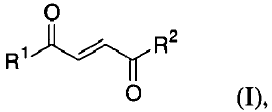

wherein R¹ and R² are independently selected from OH, O⁻, (C₁₋₆)alkoxy, or a pharmaceutically acceptable salt thereof. (C₁₋₆)alkoxy can be chosen from, for example, (C₁₋₅)alkoxy, (C₁₋₄)alkoxy, (C₁₋₃)alkoxy, ethoxy, methoxy, (C₂₋₃)alkoxy, (C₂₋₄)alkoxy, (C₂₋₅)alkoxy, and (C₁₋₆)alkoxy. In some embodiments of the compounds of Formula I, the pharmaceutically acceptable salt is a salt of a metal (M) cation, wherein M can be an alkali, alkaline earth, or transition metal such as Li, Na, K, Ca, Zn, Sr, Mg, Fe, or Mn. In nonlimiting illustrative embodiments, the compound of Formula I is dimethyl fumarate (R¹ is CH₃ and R² is CH₃) or monomethyl fumarate (R¹ is CH₃ and R² is O⁻ or OH, e.g., a pharmaceutically acceptable salt of monomethyl fumarate, e.g., specifically, Ca-MMF).

In certain embodiments the methods of the invention provide a subject with a reduction in neurodegeneration and/or neuroinflammation. These neuroprotective effects do not necessarily eliminate all of the damages or degeneration, but rather, delay or even halt the progress of the degeneration or a prevention of the initiation of the degeneration process or an improvement to the pathology of the disorder. The methods of the invention may offer neuroprotection to any part of the nervous system, such as, the central nervous system, e.g., hippocampus, cerebellum, spinal cord, cortex (e.g., motor or somatosensory cortex), striatum, basal forebrain (cholenergic neurons), ventral mesencephalon (cells of the substantia nigra), and the locus ceruleus (neuroadrenaline cells of the central nervous system).

In some embodiments, the at least one compound or pharmaceutically acceptable salt thereof is administered in an amount and for a period of time sufficient to reduce at least one of neurodegeneration and neuroinflammation in the subject. In some embodiments, the at least one compound is administered in an amount and for a period of time sufficient to reduce astrogliosis in the subject. In some embodiments, the at least one compound or pharmaceutically acceptable salt thereof is administered in an amount and for a period of time sufficient to provide neuroprotection to the subject.

Methods of the invention may include treating the subject with a therapeutically effective amount of at least one compound chosen from DMF and MMF. For DMF or MMF, the therapeutically effective amount can range from about 1 mg/kg to about 50 mg/kg (e.g., from about 2.5 mg/kg to about 20 mg/kg or from about 2.5 mg/kg to about 15 mg/kg). Effective doses will also vary, as recognized by those skilled in the art, dependent on route of administration, excipient usage, and the possibility of co-usage with other therapeutic treatments including use of other therapeutic agents. For example, an effective dose of DMF or MMF to be administered to a subject, for example orally, can be from about 0.1 g to about 1 g per day, for example, from about 200 mg to about 800 mg per day (e.g., from about 240 mg to about 720 mg per day; or from about 480 mg to about 720 mg per day; or about 720 mg per day). For example, 720 mg per day may be administered in separate administrations of 2, 3, 4, or 6 equal doses.

In some embodiments of the methods 120 mg of dimethyl fumarate is present in the pharmaceutical preparation. In some embodiments of the methods the pharmaceutical preparation is administered to the patient three times per day (TID). In some embodiments of the methods the pharmaceutical preparation is administered to the patient two times per day (BID).

In some embodiments of the methods 240 mg of dimethyl fumarate is present in the pharmaceutical preparation. In some embodiments of the methods the pharmaceutical preparation is administered to the patient three times per day (TID). In some embodiments of the methods the pharmaceutical preparation is administered to the patient two times per day (BID).

In some embodiments of the methods the pharmaceutical preparation is administered at least one hour before or after food is consumed by the patient.

In some embodiments of the methods administering the pharmaceutical preparation further comprises administering to the patient a first dose of the pharmaceutical preparation for a first dosing period; and administering to the patient a second dose of the pharmaceutical preparation for a second dosing period. In some embodiments of the methods the first dosing period is at least one week. In some embodiments of the methods the first dose of the pharmaceutical preparation comprises 120 mg of dimethyl fumarate and the pharmaceutical preparation is administered to the patient three times per day (TID) for the first dosing period. In some embodiments of the methods the second dose of the first pharmaceutical preparation comprises 240 mg of dimethyl fumarate and the first pharmaceutical preparation is administered to the patient three times per day (TID) for the second dosing period. In some embodiments of the methods the second dose of the first pharmaceutical preparation comprises 240 mg of dimethyl fumarate and the first pharmaceutical preparation is administered to the patient two times per day (BID) for the second dosing period. In some embodiments of the methods, if the patient experiences flushing or a gastrointestinal disturbance during the second dosing period then the patient is administered a dose of the first pharmaceutical preparation comprising 120 mg of dimethyl fumarate three times per day (TID) for a period of from 1 week to 1 month

The therapeutic compound (e.g., DMF or MMF) can be administered by any method that permits the delivery of the compound for treatment of neurological disorders. For instance, the therapeutic compound can be administered via pills, tablets, microtablets, pellets, micropellets, capsules (e.g., containing microtablets), suppositories, liquid formulations for oral administration, and in the form of dietary supplements. The pharmaceutically acceptable compositions can include well-known pharmaceutically acceptable excipients, e.g., if the composition is an aqueous solution containing the active agent, it can be an isotonic saline, 5% glucose, or others. Solubilizing agents such as cyclodextrins, or other solubilizing agents well known to those familiar with the art, can be utilized as pharmaceutical excipients for delivery of the therapeutic compound. See, e.g., US Patent Nos. 6,509,376 and 6,436,992 for some formulations containing DMF and/or MMF. As to route of administration, the compositions can be administered orally, intranasally, transdermally, subcutaneously, intradermally, vaginally, intraaurally, intraocularly, intramuscularly, buccally, rectally, transmucosally, or via inhalation, or intravenous administration. Preferably, DMF or MMF is administered orally.

In some embodiments, a method according to the invention comprises administering orally a capsule containing a pharmaceutical preparation consisting essentially of 60-240 mg (e.g., 120 mg) of dimethyl fumarate in the form of enteric-coated microtablets. In some embodiments, the mean diameter of such microtablets is 1-5 mm, e.g., 1-3 mm or 2 mm.

The therapeutic compound can be administered in the form of a sustained or controlled release pharmaceutical formulation. Such formulation can be prepared by various technologies by a skilled person in the art. For example, the formulation can contain the therapeutic compound, a rate-controlling polymer (i.e., a material controlling the rate at which the therapeutic compound is released from the dosage form) and optionally other excipients. Some examples of rate-controlling polymers are hydroxy alkyl cellulose, hydroxypropyl alkyl cellulose (e.g., hydroxypropyl methyl cellulose, hydroxypropyl ethyl cellulose, hydroxypropyl isopropyl cellulose, hydroxypropyl butyl cellulose and hydroxypropyl hexyl cellulose), poly(ethylene)oxide, alkyl cellulose (e.g., ethyl cellulose and methyl cellulose), carboxymethyl cellulose, hydrophilic cellulose derivatives, and polyethylene glycol, and compositions as described in WO 2006/037342, WO 2007/042034, WO 2007/042035, WO 2007/006308, WO 2007/006307, and WO 2006/050730.

In some embodiments in which dimethyl fumarate is administered to a the patient the DMF is formulated in capsules containing enteric coated microtablets. This formulation is referred to herein as "BG-12" or "BG00012". The coating of the tablets is composed of different layers. The first layer is a methacrylic acid - methyl methacrylate copolymer/isopropyl alcohol solution which isolates the tablet cores from potential hydrolysis from the next applied water suspensions. Enteric coating of the tablet is then conferred by an aqueous methacrylic acid-ethyl acrylate copolymer suspension. The complete components and quantitative composition of the capsules are given in Table 1.

**Table 1**

| **Ingredients** | **Amount/capsule** | **Function** |
|---|---|---|
| | | |

| **Core Microtablets** | | |
|---|---|---|
| *Active ingredients:* | | |
| Dimethyl Fumarate* | 120.00 mg | active ingredient |
| *Excipients:* | | |
| Croscarmellose sodium | 15.00 mg | disintegrant |
| Microcrystalline Cellulose | 131.60 mg | filler |
| Magnesium stearate | 5.00mg | lubricant |
| Talcum | 19.80mg | glidant |
| Silica colloidal anhydrous | 2.60mg | glidant |
| *Mass core microtablets* | 294.00mg | |
| | | |

| **Coating Microtablets** | | |
|---|---|---|
| *Excipients:* | | |
| Triethyl Citrate** | 7.60mg | plasticizer |
| Methacrylic Acid-Methyl Methacrylate Copolymer (1:1) | 5.50mg | film coating agent |
| as | | |
| Methacrylic Acid-Methyl Methacrylate Copolymer (1:1) solution 12.5%** | (44.00 mg) | |
| Simeticone (corresponding to Simeticone Ph Eur) | 0.17 mg | anti-foam agent |
| as | (0.53 mg) | |
| Simeticone Emulsion USP** | | |
| Talcum micronised** | 13.74 mg | lubricant |
| Methacrylic acid - Ethyl Acrylate Copolymer (1:1) | 33.00 mg | film coating agent |
| as | | |
| Methacrylic acid - Ethyl Acrylate Copolymer (1:1) dispersion 30% ** | (110.00 mg) | |
| *Mass enteric coated microtablets* | 354.01 mg | |
| *Mass of gelatin capsule* | 96.00mg | |
| *Mass of filled capsule* | 450.01 mg | |

The manufacturing process and process controls include the following:
A) Active and non-active ingredients are weighed and each starting material is identified with an appropriate labelling (denomination, batch number, quantity).
B) Blending: A powder mixture containing the active ingredient dimethyl fumarate and all excipients of the core microtablets is prepared.
C) Tabletting: A rotative press is equipped with multiple-punches tools, a deduster and the powder mixture is tabletted according to the given specifications.
D) Film Coating: In accordance with commonly used film coating methods the microtablet cores are isolated by spraying an isolation solution using a film coating equipment. The isolated cores are sprayed with an enteric coating suspension in the film coating pan. The gastro-resistance of microtablets and the active ingredient content are controlled.
E) Capsule Filling: Based on microtablets active ingredient the capsules are filled with an amount corresponding to 120 mg of active ingredient per capsule. The capsule filling weight and capsule length are controlled.
F) Packaging: The capsules are packaged on a blistering machine in thermoformed PVC/PE/PVdC - Aluminium blisters.

Additional methods of synthesizing and formulating DMF and MMF are provided, for example, in the Examples at columns 5-7 of U.S. Patent No. 7,320,999, and in WO 2006/037342, WO 2007/042034, WO 2007/042035, WO 2007/006308, WO 2007/006307, and WO 2006/050730.

The invention will be further described in the following examples, which do not limit the scope of the invention described in the claims.

### METHODS

### Rat EAE:

Female Brown Norway rats (Charles River Laboratories) were immunized intradermally at the base of the tail with MOG 1-125 100 ug/rat in IFA at day 0. Day 3 DMF (BG00012) qd 5, 25, 50, 100, 200 mg/kg delivered orally as a suspension in 0.8 % HPMC. N=6 per group. Scoring 0=no disease, 1= tail paralysis, 2=hind limb weakness, 3=hind limb paralysis, 4=hind limb paralysis and forelimb weakness, 5= moribund or dead. Experimental in vivo procedures were performed in accordance with Institutional Animal Committee guidelines

### Primary Astrocyte Cultures:

Rat astrocytes from cortex, hippocampus and striatum (Lonza clontech) were cultured as described by manufacturers protocol. Limiting dilutions of DMF in DMSO were added to cultures for 6 or 24 hours and stimulated with E. Coli LPS (Sigma). RNA was prepared using QIAgen Rneasy method.

### Histology and Morphometry:

Lumbar spinal cord sections were prepared as FFPE sections for immunohistochemistry and processed on DAKO autostainer using GFAP antibody (DAKO), IBA-1 (Wako), CD3 (DAKO). Aperio Spectrum Color Deconvolution software was used for morphometric analysis.

### Expression Analysis:

Total RNA was made from snap frozen lumbar spinal cord sections using Qiagen RNeasy methods. Applied Biosciences TaqMan probes were used to amplify specific transcripts and normalized using the GAPDH housekeeping probe.

### EXAMPLE 1:

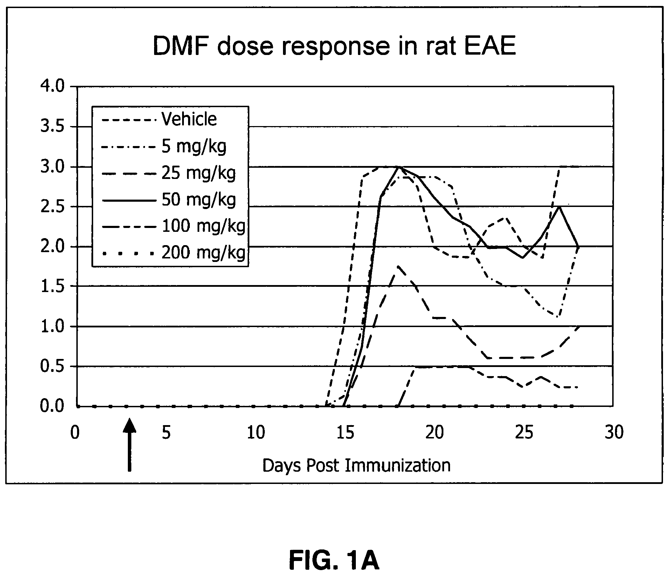

BG00012, an orally available formulation of dimethyl fumarate (DMF), is in Phase III testing for relapsing-remitting multiple sclerosis (RRMS). In Phase IIb testing, BG-12 significantly reduced gadalinium enhancing brain lesions and reduced T1 hypointense black holes. The active component of BG00012, dimethyl fumarate (DMF), was tested in rat EAE models. As shown in Figure 1A, treatment of rats with experimentally induced EAE with various doses of DMF reduced EAE symptoms in a dose-dependent manner. Treatment with 200 mg/kg DMF completely abrogated disease. (Figure 1A).

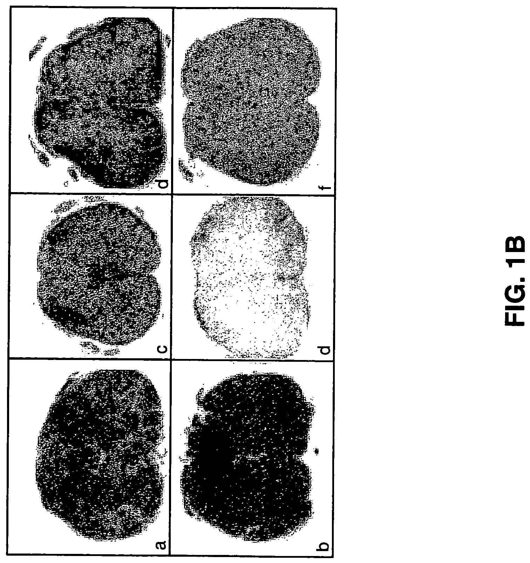

Figure 1B shows cross sections of spinal cords of rats treated with vehicle or BG00012. The treatment and staining of the panels a-f is as follows:
a. Luxol Fast Blue / vehicle
b. Luxol Fast Blue / BG00012
c. GFAP / vehicle
d. GFAP / BG00012
e. IBA1/ vehicle
f. IBA1/ BG0012

As shown in Figure 1B, activated astrocytes and microglia are markedly reduced in spinal cords treated with BG00012 and myelin is preserved.

### EXAMPLE 2

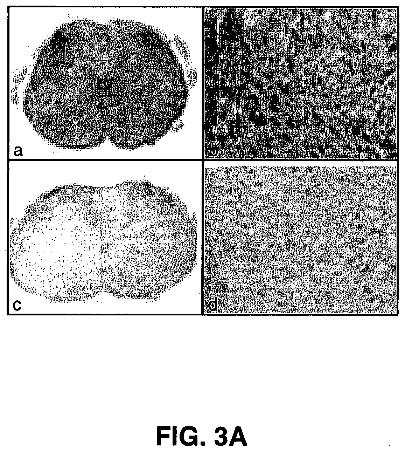

This example shows that BG00012 reduces astrocyte activation in vivo. Specifically, spinal cords from DMF treated rats have fewer activated astrocytes in they grey matter than spinal cords of rats receiving vehicle alone. Figure 3A shows cross sections of spinal cords stained with a GFAP antibody to identify activated astrocytes. Panels (a) and (b) are from a rat treated with vehicle alone at 5x and 20x magnification, respectively, while panels (c) and (d) are from a rat treated with 100 mg/kg DMF, shown at 5x and 20x magnification, respectively.

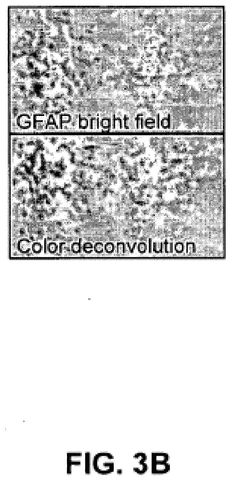

Figure 3B shows morphometric quantitation using Apeiro color deconvolution.

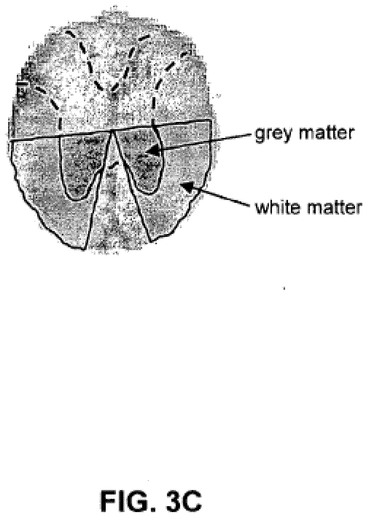

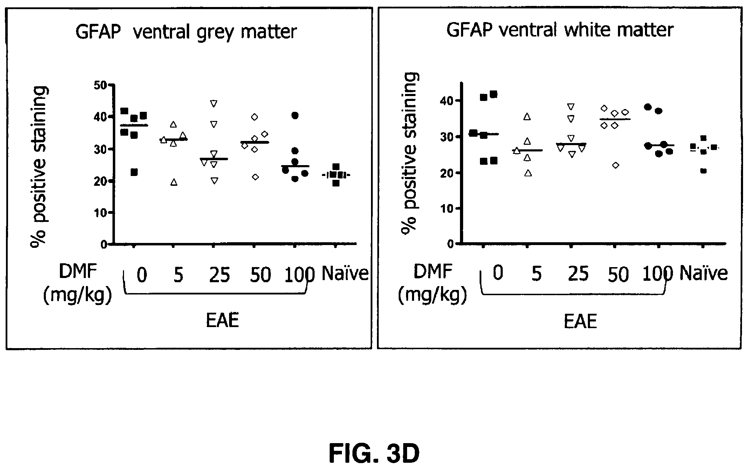

Figure 3C shows a rat spinal cord cross section with the ventral grey matter and white matter zones indicated. Those zones were selected for morphometry. Figure 3 D shows morphometric quantitation of positive GFAP staining in ventral grey and white matter.

### EXAMPLE 3

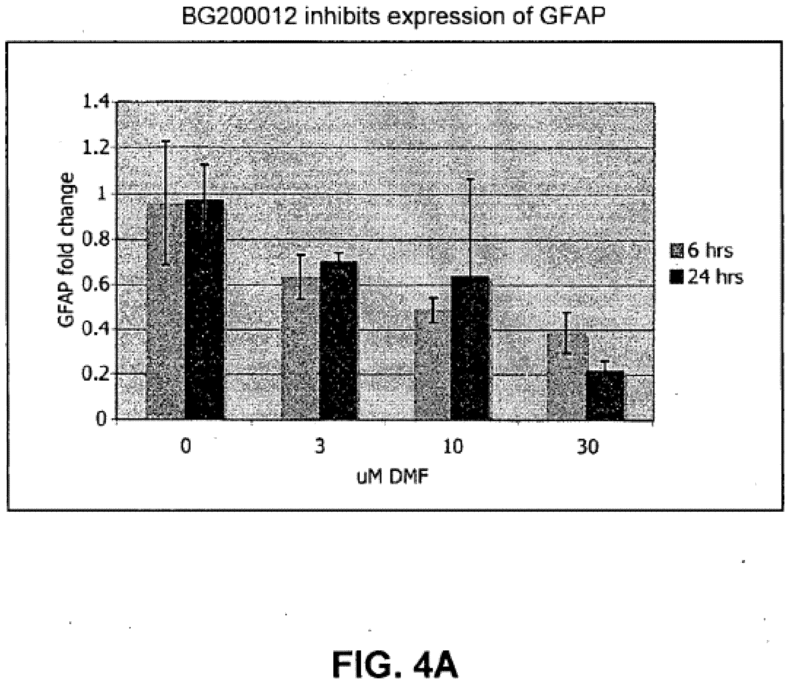

This experiment shows that BG00012 reduces activation of primary cultured astrocytes. Figure 4A shows quantitative PCR analysis of GFAP expression following DMF stimulation at the indicated concentrations for 6 hrs and 24 hrs. As shown, DMF inhibits GFAP expression in a concentration dependent manner.

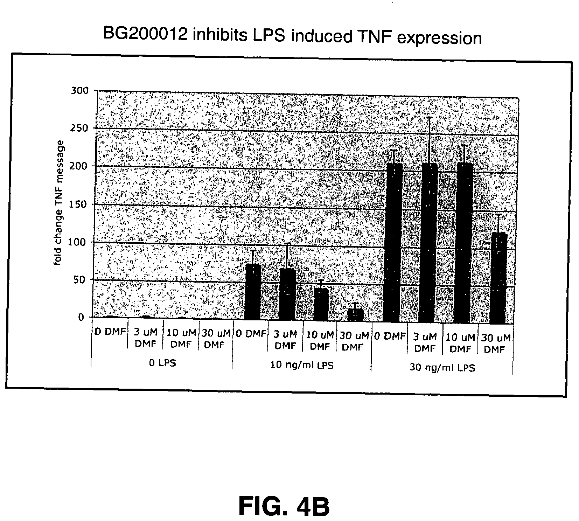

Figure 4B shows quantitative PCR analysis of TNF expression following DMF stimulation at the indicated concentration for 24 hours, with LPS added at 0, 10, and 30 ng/ml 4 hrs. prior to harvest. As shown, LPS induces TNF expression in a dose dependent manner and that induced TNF expression is repressed by DMF in a dose dependent manner.

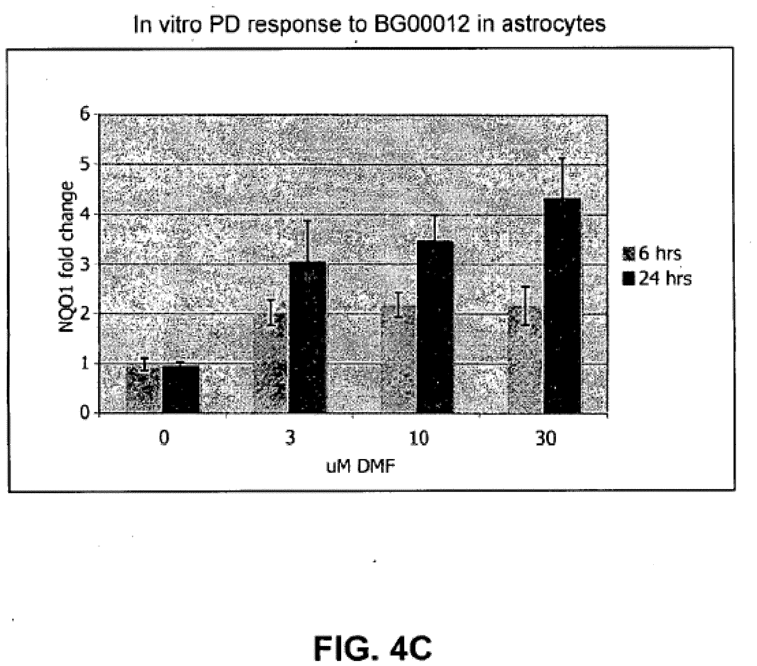

Figure 4C shows quantitative PCR analysis of NQO1 expression following DMF stimulation at the indicated concentrations for 6 hrs and 24 hrs. As shown, DMF induces NQO1 expression in a concentration dependent manner. It is also apparent from the data that NQO1 is induced at a higher level by exposure to DMF for 24 hrs compared to induction by a 6 hour exposure.

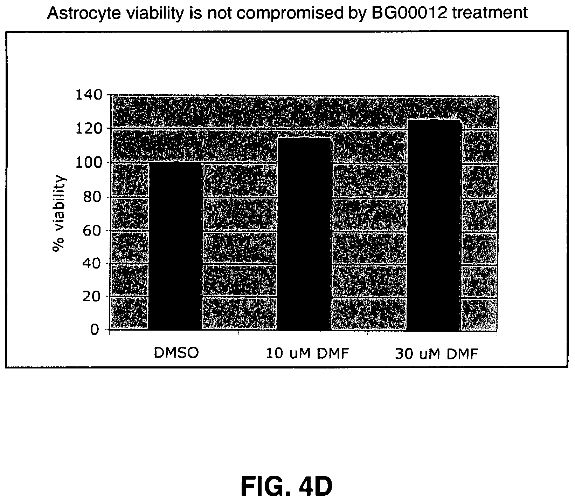

Finally, Figure 4D shows the results of an MTT assay after 24 hrs of BG00012 (DMF) or vehicle (DMSO) treatment of astrocytes. The data indicate that Astrocyte viability is not compromised by BG00012 treatment.

### EXAMPLE 4

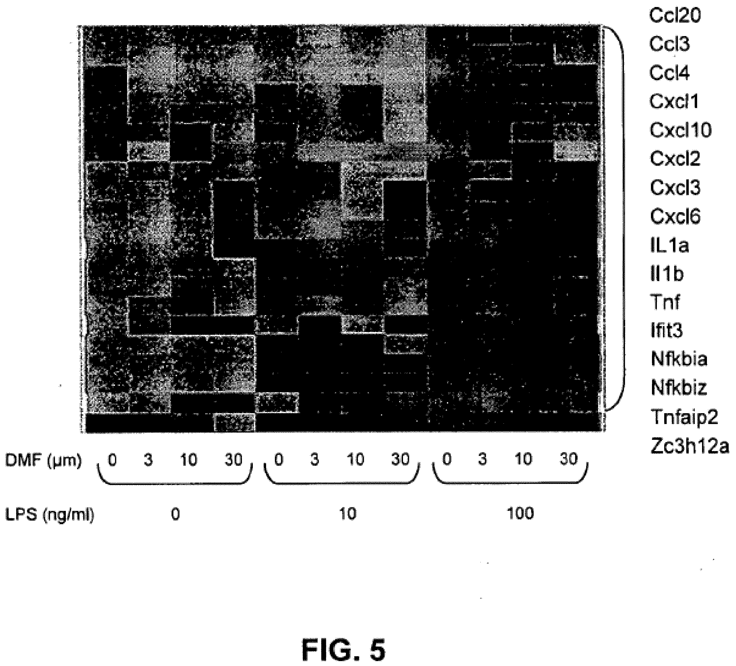

Figure 5 shows that BG00012 inhibits inflammatory cytokines and pro-inflammatory signaling induced by LPS stimulation of primary astrocytes. Primary astrocytes were treated with LPS at 0, 10, and 100 ng/ml, as indicated, and also treated with DMF at 0, 3, 10, or 30 µM as indicated. The magnitude of expression of each gene under each condition was scaled from 0 to 1, so that differences in color represent changes in expression of each gene as the conditions varied. The darkest green color represents no detectable expression (0), while the brightest red color represents the highest expression measured for that gene (1). Generally speaking, the data show that expression of these genes is increased by LPS treatment, with all markers showing induction by 100 ng/ml LPS treatment. The induced expression of many of the markers was suppressed by DMF treatment. In particular, strongly suppressed induction of Ccl20, Ccl3, Ccl4, Cxcll, Cxcl10, Cxcl2, Cxcl3, Cxcl6, IL1a, II1b, Tnf, Ifit3, and Zc3h12a.

### EXAMPLE 5

This example provides data indicating that DMF (BG00012) can inhibit astrogliosis and microglial activation associated with chronic relapsing EAE (crEAE) in rats.

crEAE was induced by intradermal MOG/IFA immunization in Brown Norway rats. BG00012 was administered orally at a daily interval beginning three days after immunization. BG00012 reduced average clinical scores of disease in all treated groups. For the 100mg/kg treatment group, average disease score at day 28 was 0.71 (n=6, SD=1.17) compared to 2.29 (n=6, SD=1.29) for the vehicle group. Immunohistochemistry of lumbar spinal cord sections showed decreased staining of GFAP, a marker for activated astrocytes, and IBA-1, a marker for activated microglia.

Quantitative PCR of mRNA from spinal cords revealed 52% and 54% decreases in IBA-1 and GFAP mRNAs, respectively, in BG00012 treated group compared to the vehicle treated group.

Direct effects of BG00012 on specific astrocytic and microglial cells were tested in vitro using primary rat astrocytes and RAW264.7 macrophage cells. LPS stimulation in the presence of BG00012 resulted in 77% and 59% reduction in TNF-a mRNA in astrocytes and macrophages, respectively. Global gene expression profiling of LPS stimulated cells showed that BG00012 can inhibit many pro-inflammatory gene products in both cell types.

These findings indicate that suppression of reactive gliosis and inhibition of macrophage function may contribute to the therapeutic effect of BG00012 as a part of its dual anti-inflammatory and CNS neuroprotective mechanism of action.

The data reported herein indicate that BG00012 inhibits disease in relapsing rat EAE. Histological analysis has shown decreased levels of astrocyte activation markers in BG00012-treated spinal cords. In vitro data suggest that BG00012 can directly inhibit activation of astrocytes. Finally, pro-Inflammatory gene expression is reduced following BG00012 treatment in LPS stimulated primary astrocytes. These findings point to a role for BG00012 in suppression of reactive gliosis and dual anti-inflammatory and CNS neuroprotective mechanisms of action.

### EXAMPLE 6

This Example analyzes the effect of MMF on cultured astrocytes. Specifically, the results show that MMF treatment upregulates cellular reduction potential, reduces H₂O₂-induced Ca⁺⁺ mobilization, and reduces H₂O₂-induced cellular death.

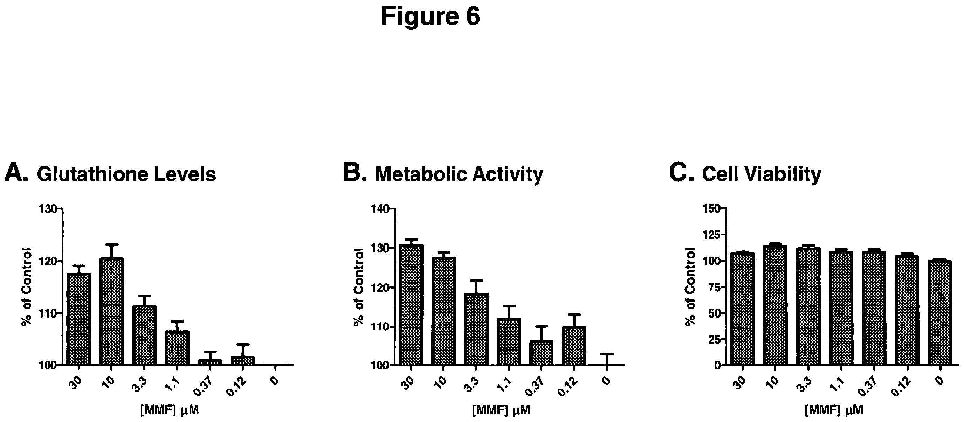

Primary cultures of human spinal cord astrocytes were treated for 24 hr with a titration of MMF or DMSO as a diluent control. Following 24 hr incubation with compound, cells were washed 1X with growth media and incubated with the cell-permeant substrate monochlorobimane. When bound to glutathione this substrate increases fluorescence. A clear concentration-dependent increase in cellular glutathione levels were observed upon treatment with MMF. Figure 6A. Similar MMF treated cells were also incubated with the cell permeant substrate resazurin, which increases fluorescence upon reduction to resorufin by cellular redox mechanisms and is used as a measure of cellular metabolic activity (CellTiter-Blue assay). This assay demonstrates a similar result as in (Fig. 6A), in that there is a clear MMF concentration-dependent increase in the ability of treated cells to reduce the substrate. Figure 6B. To ensure the increases in glutathione and metabolic activity were not simply due to cellular proliferation in response to MMF treatment, parallel dishes of cells were incubated with calcein AM. Cellular esterases cleave this molecule to generate a fluorescent metabolite, which provides a measure of cell viability and relative total cell numbers. No substantial concentration-dependent changes were observed. Figure 6C. Taken together these data suggest MMF treatment upregulates an antioxidant response in cultured human spinal cord astrocytes, which may offer neuroprotective benefit upon oxidative challenge.

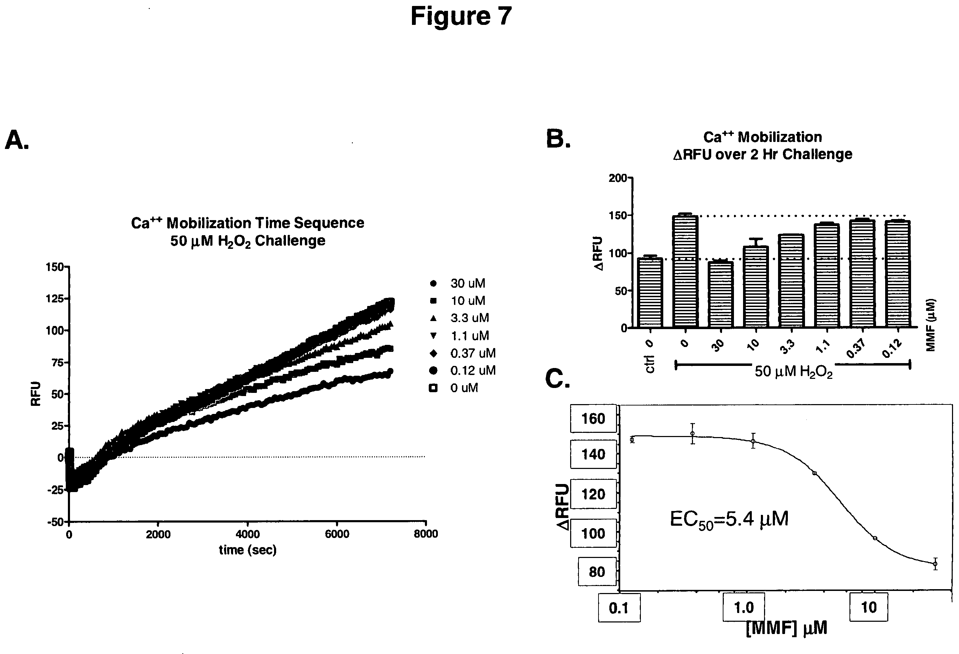

In another experiment, primary cultures of human spinal cord astrocytes were treated for 24 hr with a titration of MMF or DMSO as a diluent control. Following 24 hr incubation with compound, cells were washed IX with HBSS and incubated with Calcium4 (Molecular Devices) loading dye. This dye permeates into cells and increases fluorescence upon binding of free intracellular Ca⁺⁺. Cells were then challenged with 50 mM H₂O₂ and monitored for changes in fluorescence . Figure 7A shows raw fluorescence traces from cells indicated MMF reduced mobilization of intracellular Ca++ in a dose-dependent fashion. Figure 7B shows quantitation of the change in fluorescence intensity over basline (DRFU) demonstrated that 30 mM MMF reduced accumulation of Ca⁺⁺ to background levels (compared to no H₂O₂ control), and protection against the H₂O₂ challenge is dose dependent. Fitting this data with non-linear regression reveals EC₅₀ = 5.4 mM. Figure 7C. These data suggest MMF is able to suppress release of intracelluar Ca++, which may offer neuroprotective benefit by preventing initiation of downstream apoptotic cascades.

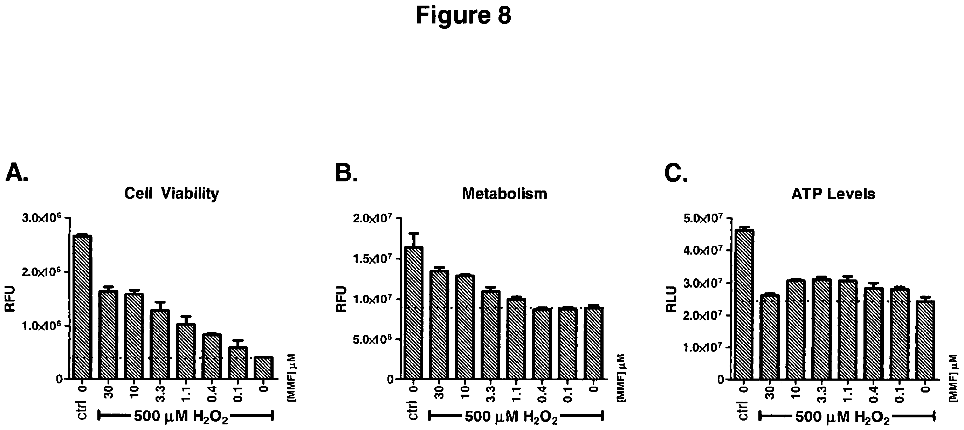

In another experiment, primary cultures of human spinal cord astrocytes were treated for 24 hr with a titration of MMF or DMSO as a diluent control. Following 24 hr incubation with compound, cells were washed IX with HBSS and challenged with 500 mM H₂O₂ for two hours, then washed in normal growth media, then incubated for an additional 24 hours. Figure 8A shows results of using the same calcein AM technique as in Figure 6 to monitor cell viability, a significant decrease was observed with transient 500 mM H₂O₂ treatment followed by a 24 hour recovery period. This loss of viability is attenuated by MMF in a concentration-dependent manner. Figure 8B shows that similar effects were observed on cellular metabolism, as measured by cell-dependent reduction of the substrate resazurin. Figure 8C shows somewhat more modest MMF-dependent protective effects were observed by examining cellular ATP levels, although a clear concentration-dependent response was observed. Interestingly, the highest concentration of MMF appeared to reduce cellular ATP levels. Taken together, these data suggest that MMF triggers a response in human spinal cord astrocytes that is neuroprotective against oxidative stress.

### EXAMPLE 7

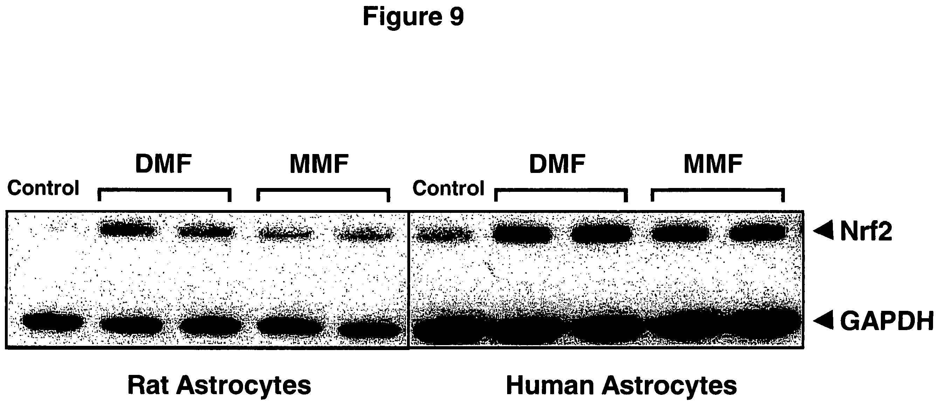

Total cell lysates were prepared from the astrocyte cultures treated as indicated above. Nrf2 was detected by Western blotting. GAPDH was included as a housekeeping protein control. The results shown in Figure 9 show that DMF and MMF increase levels of Nrf2 in primary rat and human astrocytes.

### EXAMPLE 8

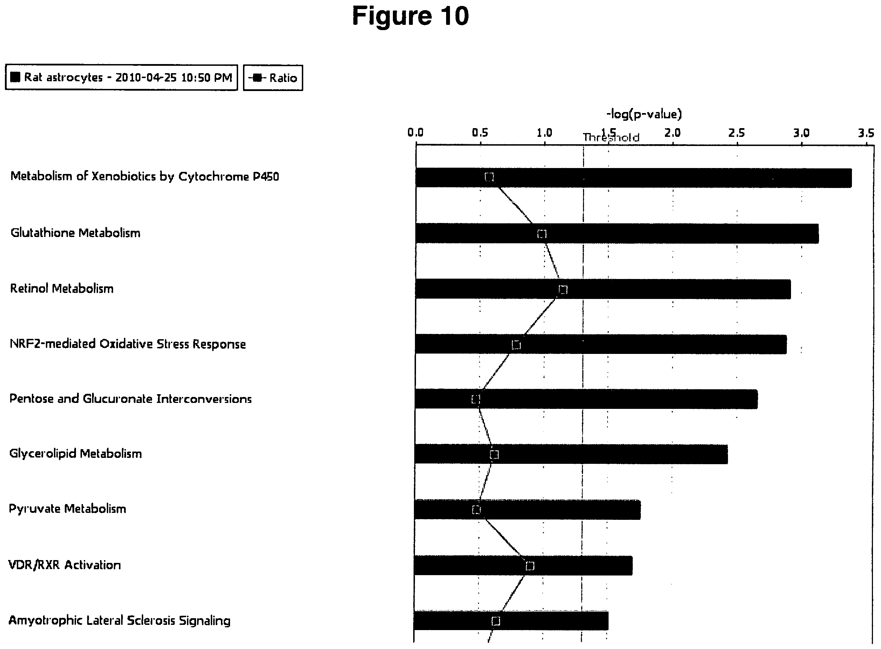

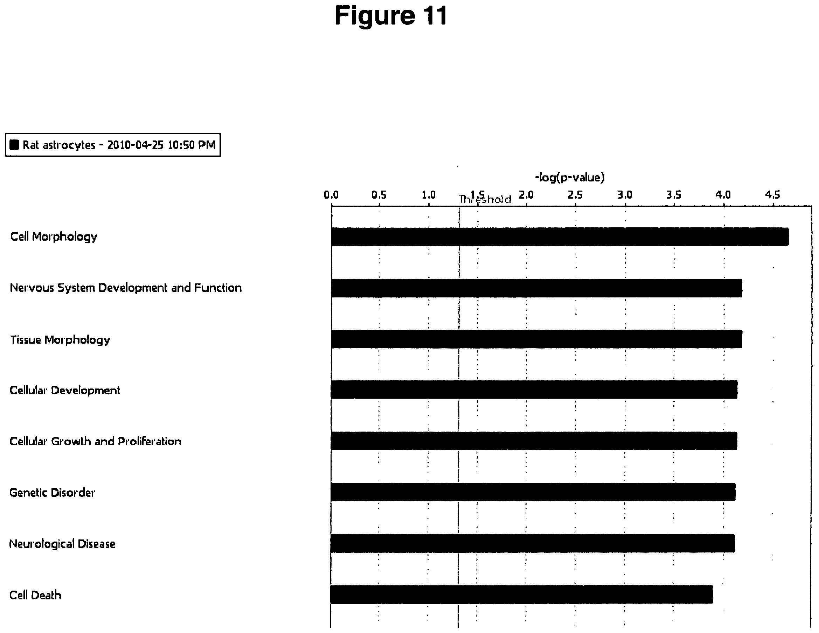

Global analysis of gene expression in astrocytes treated with DMF was performed using the Affymetrix GeneChip technology. Genes affected by DMF were identified as transcripts whose levels were significantly (p<10⁻⁷) increased in DMF-treated cells compared to the untreated astrocytes. The resulting gene list was annotated using the Ingenuity IPA database. As shown in Figure 10, DMF activates expression of Nrf2 target genes, including genes known to regulate glutathione metabolism. As shown in Figure 11, modulation of global gene expression by DMF indicates effects on astrocyte functions related to nervous system development, function, and disease. Specific genes involved in cytoprotection and glutathione metabolism, identified in this assay, include, but are not limited to, Gsta2, Gsta3, Gclc, Ggtl, Txnrd1, Srxnl, Sqstml, and Nqol.

### EXAMPLE 9

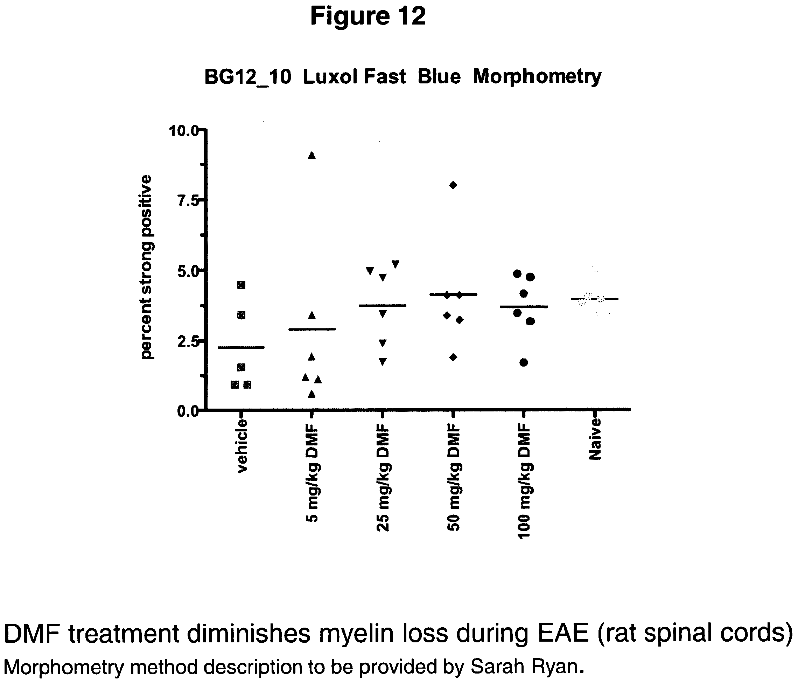

As shown in Figure 12, DMF treatment diminishes myelin loss during EAE in rat spinal cords. Morphometry analysis of EAE spinal cords was performed with Aperio software. Histological alterations were quantified with Aperio Image Scope software v10.1. The Color Deconvolution algorithm was used to select intensity thresholds either for brown DAB staining (GFAP and IBA-1 Immunostains) or for bright turquoise blue staining (Luxol Fast Blue). Positive staining was determined by the percent of strong positive stained pixels for each intensity threshold. Three lumbar spinal cord sections were quantitated per rat and the values for percent strong positive pixels were averaged for the final intensity value.

In view of the foregoing, it will be appreciated that the invention described herein inter alia relates to the following items:
1. A method of treating a subject having a condition characterized by at least one symptom chosen from neurodegeneration and neuroinflammation, the method comprising administering to the subject a therapeutically effective amount of at least one compound of Formula I: 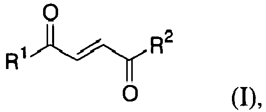
   wherein R¹ and R² are independently selected from OH, O⁻, and (C₁₋₆)alkoxy, provided that at least one of R¹ and R² is (C₁₋₆)alkoxy, or a pharmaceutically acceptable salt thereof,
   wherein the administration to the subject of the therapeutically effective amount of the at least one compound of Formula I, or a pharmaceutically acceptable salt thereof, results in the supression of expression in the subject of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a.
2. The method of item 1, wherein the condition characterized by at least one symptom chosen from neurodegeneration and neuroinflammation is further characterized by increased expression of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a.
3. The method of item 1 or 2, wherein the administration to the subject of the therapeutically effective amount of the at least one compound of Formula I results in upregulation of expression in the subject of at least one gene selected from Gsta2, Gsta3, Gclc, Ggt1, Txnrd1, Srxn1, Sqstm1, and Nqo1.
4. The method of item 1, wherein the at least one compound is formulated as a pharmaceutical composition comprising the at least one compound and at least one pharmaceutically acceptable vehicle chosen from carriers, adjuvants, and excipients.
5. The method of any one of items 1-4, wherein the at least one compound is chosen from dimethyl fumarate and monomethyl fumarate.
6. The method of any one of items 1-4, wherein the only active agent administered to the subject is dimethyl fumarate (DMF).
7. The method of any one of items 1-4, wherein the only active agent administered to the subject is monomethyl fumarate (MMF).
8. The method of any one of items 1-4, wherein the only active agents administered to the subject are DMF and MMF.
9. The method of any one of items 1 to 8, wherein the at least one compound is administered in an amount and for a period of time sufficient to reduce at least one of neurodegeneration and neuroinflammation in the subject.
10. The method of any one of items 1 to 9, wherein the condition characterized by at least one symptom chosen from neurodegeneration and neuroinflammation is selected from Adrenal Leukodystrophy (ALD), Alcoholism, Alexander's disease, Alper's disease, Alzheimer's disease, Amyotrophic lateral sclerosis (Lou Gehrig's Disease), Ataxia telangiectasia, Batten disease (also known as Spielmeyer-Vogt-Sjögren-Batten disease), Bovine spongiform encephalopathy (BSE), Canavan disease, Cerebral palsy, Cockayne syndrome, Corticobasal degeneration, Creutzfeldt-Jakob disease, Familial Fatal Insomnia, Frontotemporal lobar degeneration, Huntington's disease, HIV-associated dementia, Kennedy's disease, Krabbe's disease, Lewy body dementia, Neuroborreliosis, Machado-Joseph disease (Spinocerebellar ataxia type 3), Multiple System Atrophy, Multiple sclerosis, Narcolepsy, Niemann Pick disease, Parkinson's disease, Pelizaeus-Merzbacher Disease, Pick's disease, Primary lateral sclerosis,
   Prion diseases, Progressive Supranuclear Palsy, Refsum's disease, Sandhoff disease, Schilder's disease, Subacute combined degeneration of spinal cord secondary to Pernicious Anaemia, Spielmeyer-Vogt-Sjogren-Batten disease (also known as Batten disease), Spinocerebellar ataxia, Spinal muscular atrophy, Steele-Richardson-Olszewski disease, Tabes dorsalis, Toxic encephalopathy, LHON (Leber's Hereditary optic neuropathy), MELAS (Mitochondrial Encephalomyopathy; Lactic Acidosis; Stroke), MERRF (Myoclonic Epilepsy; Ragged Red Fibers), PEO (Progressive External Opthalmoplegia), Leigh's Syndrome, MNGIE (Myopathy and external ophthalmoplegia; Neuropathy; Gastro-Intestinal; Encephalopathy), Kearns-Sayre Syndrome (KSS), NARP, Hereditary Spastic Paraparesis, Mitochondrial myopathy, and Friedreich Ataxia.
11. The method of item 10, wherein the condition characterized by at least one of neurodegeneration and neuroinflammation is Multiple Sclerosis (MS).
12. The method of any one of items 1 to 9, wherein the subject does not have MS.
13. A method of reducing astrogliosis in a subject having a condition characterized by increased astrogliosis, the method comprising administering to the subject a therapeutically effective amount of at least one compound of Formula I: 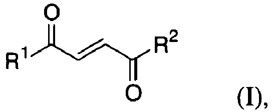 wherein R¹ and R² are independently selected from OH, O⁻, and (C₁₋₆)alkoxy, or a pharmaceutically acceptable salt thereof.
14. The method of item 13, wherein the administration to the subject of the therapeutically effective amount of the at least one compound of Formula I, or a pharmaceutically acceptable salt thereof, results in the supression of expression in the subject of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a.
15. The method of item 13 or 14, wherein the administration to the subject of the therapeutically effective amount of the at least one compound of Formula I results in upregulation of expression in the subject of at least one gene selected from Gsta2, Gsta3, Gclc, Ggt1, Txnrd1, Srxn1, Sqstm1, and Nqo1.
16. The method of any one of items 13-15, wherein the at least one compound is formulated as a pharmaceutical composition comprising the at least one compound and at least one pharmaceutically acceptable vehicle chosen from carriers, adjuvants, and excipients.
17. The method of any one of items 13-16, wherein the at least one compound is formulated as a pharmaceutical composition comprising the at least one compound and at least one pharmaceutically acceptable vehicle chosen from carriers, adjuvants, and excipients.
18. The method of any one of items 13-17, wherein the at least one compound is chosen from dimethyl fumarate and monomethyl fumarate.
19. The method of any one of items 13-17, wherein the only active agent administered to the subject is dimethyl fumarate (DMF).
20. The method of any one of items 13-17, wherein the only active agent administered to the subject is monomethyl fumarate (MMF).
21. The method of any one of items 13-17, wherein the only active agents administered to the subject are DMF and MMF.
22. The method of any one of items 13-21, wherein the at least one compound is administered in an amount and for a period of time sufficient to reduce at least one of neurodegeneration and neuroinflammation in the subject.
23. The method of any one of items 13-22, wherein the condition characterized by increased astrogliosis is selected from Adrenal Leukodystrophy (ALD), Alcoholism, Alexander's disease, Alper's disease, Alzheimer's disease, Amyotrophic lateral sclerosis (Lou Gehrig's Disease), Ataxia telangiectasia, Batten disease (also known as Spielmeyer-Vogt-Sjögren-Batten disease), Bovine spongiform encephalopathy (BSE), Canavan disease, Cerebral palsy, Cockayne syndrome, Corticobasal degeneration, Creutzfeldt-Jakob disease, Familial Fatal Insomnia, Frontotemporal lobar degeneration, Huntington's disease, HIV-associated dementia, Kennedy's disease, Krabbe's disease, Lewy body dementia, Neuroborreliosis, Machado-Joseph disease (Spinocerebellar ataxia type 3), Multiple System Atrophy, Multiple sclerosis, Narcolepsy, Niemann Pick disease, Parkinson's disease, Pelizaeus-Merzbacher Disease, Pick's disease, Primary lateral sclerosis, Prion diseases, Progressive Supranuclear Palsy, Refsum's disease, Sandhoff disease, Schilder's disease, Subacute combined degeneration of spinal cord secondary to Pernicious Anaemia, Spielmeyer-Vogt-Sjogren-Batten disease (also known as Batten disease), Spinocerebellar ataxia, Spinal muscular atrophy, Steele-Richardson-Olszewski disease, Tabes dorsalis, Toxic encephalopathy, LHON (Leber's Hereditary optic neuropathy), MELAS (Mitochondrial Encephalomyopathy; Lactic Acidosis; Stroke), MERRF (Myoclonic Epilepsy; Ragged Red Fibers), PEO (Progressive External Opthalmoplegia), Leigh's Syndrome, MNGIE (Myopathy and external ophthalmoplegia; Neuropathy; Gastro-Intestinal; Encephalopathy), Kearns-Sayre Syndrome (KSS), NARP, Hereditary Spastic Paraparesis, Mitochondrial myopathy, and Friedreich Ataxia.
24. The method of item 23, wherein the condition characterized by at least one of neurodegeneration and neuroinflammation is Multiple Sclerosis (MS).
25. The method of any one of items 13-22, wherein the subject does not have MS.
26. A method of identifying a compound as a candidate neuroprotection agent, the method comprising
   a) inducing at least one of neurodegeneration and neuroinflammation in a target cell, tissue, or mammal,
   b) measuring expression of at least one marker of at least one of neurodegeneration and neuroinflammation in the target cell, tissue, or mammal in the presence of the compound, and
   c) measuring expression of at least one marker of at least one of neurodegeneration and neuroinflammation in the target cell, tissue, or mammal in the absence of the compound, wherein, if the expression of at least one marker of at least one of neurodegeneration and neuroinflammation is reduced in the presence of the compound relative to its expression in the absence of the compound, the compound is identified as a candidate neuroprotection agent.
27. The method of item 26, further comprising:
   d) measuring astrogliosis in the presence of the at least one compound, and
   e) measuring astrogliosis in the absence of the at least once compound,
   wherein, astrogliosis is reduced in the presence of the compound relative to the level of astrogliosis in the absence of the compound.
28. The method of item 26, wherein the at least one marker is the expression level of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a.
29. A method of providing neuroprotection to a subject in need thereof, the method comprising administering to the subject a therapeutically effective amount of at least one compound of Formula I: 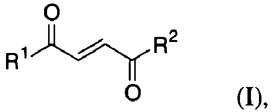 wherein R¹ and R² are independently selected from OH, O⁻, and (C₁₋₆)alkoxy, provided that at least one of R¹ and R² is (C₁₋₆)alkoxy, or a pharmaceutically acceptable salt thereof,
   wherein the administration to the subject of the therapeutically effective amount of the at least one compound of Formula I, or a pharmaceutically acceptable salt thereof, results in the supression of expression in the subject of at least one gene selected from Ccl20, Ccl3, Ccl4, Cxcl1, Cxcl10, Cxcl2, Cxcl3, Cxcl6, IL1a, II1b, Tnf, Ifit3, Nfkbia, Nfkbiz, Tnfaip2, and Zc3h12a.
30. The method of item 29, wherein the condition characterized by at least one symptom chosen from neurodegeneration and neuroinflammation is further characterized by increased expression of at least one gene selected from Ccl20, Ccl3, Ccl4, Cxcll, Cxcl10, Cxcl2, Cxcl3, Cxcl6, IL1a, II1b, Tnf, Ifit3, Nfkbia, Nfkbiz, Tnfaip2, and Zc3h12a.
31. The method of item 29 or 30, wherein the administration to the subject of the therapeutically effective amount of the at least one compound of Formula I results in upregulation of expression in the subject of at least one gene selected from Gsta2, Gsta3, Gclc, Ggt1, Txnrd1, Srxn1, Sqstm1, and Nqo1.
32. The method of any one of items 29-31, wherein the at least one compound is formulated as a pharmaceutical composition comprising the at least one compound and at least one pharmaceutically acceptable vehicle chosen from carriers, adjuvants, and excipients.
33. The method of any one of items 29-32, wherein the at least one compound is chosen from dimethyl fumarate and monomethyl fumarate.
34. The method of any one of items 29-32, wherein the only active agent administered to the subject is dimethyl fumarate (DMF).
35. The method of any one of items 29-32, wherein the only active agent administered to the subject is monomethyl fumarate (MMF).
36. The method of any one of items 29-32, wherein the only active agents administered to the subject are DMF and MMF.
37. The method of any one of items 29 to 36, wherein the at least one compound is administered in an amount and for a period of time sufficient to reduce at least one of neurodegeneration and neuroinflammation in the subject.
38. The method of any one of items 29 to 37, wherein the condition characterized by at least one symptom chosen from neurodegeneration and neuroinflammation is selected from Adrenal Leukodystrophy (ALD), Alcoholism, Alexander's disease, Alper's disease, Alzheimer's disease, Amyotrophic lateral sclerosis (Lou Gehrig's Disease), Ataxia telangiectasia, Batten disease (also known as Spielmeyer-Vogt-Sjögren-Batten disease), Bovine spongiform encephalopathy (BSE), Canavan disease, Cerebral palsy, Cockayne syndrome, Corticobasal degeneration, Creutzfeldt-Jakob disease, Familial Fatal Insomnia, Frontotemporal lobar degeneration, Huntington's disease, HIV-associated dementia, Kennedy's disease, Krabbe's disease, Lewy body dementia, Neuroborreliosis, Machado-Joseph disease (Spinocerebellar ataxia type 3), Multiple System Atrophy, Multiple sclerosis, Narcolepsy, Niemann Pick disease, Parkinson's disease, Pelizaeus-Merzbacher Disease, Pick's disease, Primary lateral sclerosis, Prion diseases, Progressive Supranuclear Palsy, Refsum's disease, Sandhoff disease, Schilder's disease, Subacute combined degeneration of spinal cord secondary to Pernicious Anaemia, Spielmeyer-Vogt-Sjogren-Batten disease (also known as Batten disease), Spinocerebellar ataxia, Spinal muscular atrophy, Steele-Richardson-Olszewski disease, Tabes dorsalis, Toxic encephalopathy, LHON (Leber's Hereditary optic neuropathy), MELAS (Mitochondrial Encephalomyopathy; Lactic Acidosis; Stroke), MERRF (Myoclonic Epilepsy; Ragged Red Fibers), PEO (Progressive External Opthalmoplegia), Leigh's Syndrome, MNGIE (Myopathy and external ophthalmoplegia; Neuropathy; Gastro-Intestinal; Encephalopathy), Kearns-Sayre Syndrome (KSS), NARP, Hereditary Spastic Paraparesis, Mitochondrial myopathy, and Friedreich Ataxia.
39. The method of item 38, wherein the condition characterized by at least one of neurodegeneration and neuroinflammation is Multiple Sclerosis (MS).
40. The method of any one of items 29 to 37, wherein the subject does not have MS.

Additionally, the invention described herein also relates to the following items:
1. Dimethyl fumarate or a pharmaceutically acceptable salt thereof for use in the treatment of a subject having multiple sclerosis, wherein the treatment comprises administering to the subject a first dose of a pharmaceutical preparation for a first dosing period and administering to the subject a second dose of the pharmaceutical preparation for a second dosing period, wherein the second dose of the pharmaceutical preparation comprises 240 mg dimethyl fumarate and is administered to the subject two times per day (BID) for the second dosing period.
2. The dimethyl fumarate for the use of item 1, wherein the first dosing period is at least one week.
3. The dimethyl fumarate for the use of item 2, wherein the first dosing period is one week.
4. The dimethyl fumarate for the use of any one of items 1 to 3, wherein the pharmaceutical preparation is administered at least one hour before or after food is consumed by the subject.
5. The dimethyl fumarate for the use of any one of items 1 to 4, wherein the only active agent administered to the subject is dimethyl fumarate.
6. The dimethyl fumarate for the use of any one of items 1 to 5, wherein the only active agents administered to the subject are dimethyl fumarate and monomethyl fumarate.
7. The dimethyl fumarate for the use of any one of items 1 to 6, wherein the pharmaceutical preparation is in the form of a pill, a tablet, a microtablet, a pellet, a micropellet, a capsule, a capsule containing a microtablet or a liquid formulation.
8. The dimethyl fumarate for the use of any one of items 1 to 7, wherein the pharmaceutical preparation is a capsule consisting essentially of 60-240 mg of dimethyl fumarate in the form of enteric coated microtablets.

## Claims

1. At least one compound of Formula I, or a pharmaceutically acceptable salt thereof, for use in treating a subject having Friedreich ataxia, wherein Formula I is: 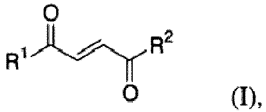 wherein R¹ and R² are independently selected from OH, O⁻, and (C₁₋₆)alkoxy, provided that at least one of R¹ and R² is (C₁₋₆)alkoxy.

2. The at least one compound of Formula I, or a pharmaceutically acceptable salt thereof, for use as in claim 1, wherein the at least one compound of Formula I, or a pharmaceutically acceptable salt thereof, is dimethyl fumarate and/or monomethyl fumarate.

3. The at least one compound of Formula I, or a pharmaceutically acceptable salt thereof, for use as in claim 2, wherein dimethyl fumarate and/or monomethyl fumarate is/are the only active agent(s) administered to the subject.

4. The at least one compound of Formula I, or a pharmaceutically acceptable salt thereof, for use as in claim 2, wherein dimethyl fumarate is the only active agent administered to the subject.

5. The at least one compound of Formula I, or a pharmaceutically acceptable salt thereof, for use as in claim 2, wherein monomethyl fumarate is the only active agent administered to the subject.

6. The at least one compound of Formula I, or a pharmaceutically acceptable salt thereof, for use as in any one of claims 1 to 5, wherein the at least one compound of Formula I, or a pharmaceutically acceptable salt thereof, is administered orally.

7. The at least one compound of Formula I, or a pharmaceutically acceptable salt thereof, for use as in claim 6, wherein the at least one compound of Formula I, or a pharmaceutically acceptable salt thereof, is administered in the form of a pill, a tablet, a microtablet, a pellet, a micropellet, a capsule, a capsule containing microtablets, or a liquid formulation for oral administration.

8. A pharmaceutical composition for use in treating a subject having Friedreich ataxia, the composition comprising:
(a) at least one compound of Formula I, or a pharmaceutically acceptable salt thereof, wherein Formula I is: 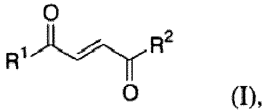 wherein R¹ and R² are independently selected from OH, O⁻, and (C₁₋₆)alkoxy, provided that at least one of R¹ and R² is (C₁₋₆)alkoxy, and
(b) one or more pharmaceutically acceptable excipients.

9. The pharmaceutical composition for use as in claim 8, wherein the at least one compound of Formula I, or a pharmaceutically acceptable salt thereof, is dimethyl fumarate and/or monomethyl fumarate

10. The pharmaceutical composition for use as in claim 9, wherein the composition comprises dimethyl fumarate and/or monomethyl fumarate as the only active agent(s) and one or more pharmaceutically acceptable excipients.

11. The pharmaceutical composition for use as in claim 9, wherein the composition comprises dimethyl fumarate as the only active agent and one or more pharmaceutically acceptable excipients.

12. The pharmaceutical composition for use as in claim 9, wherein the composition comprises monomethyl fumarate as the only active agent and one or more pharmaceutically acceptable excipients.

13. The pharmaceutical composition for use as in any one of claims 8 to 12, wherein the composition is administered orally.

14. The pharmaceutical composition for use as in claim 13, wherein the composition is administered in the form of a pill, a tablet, a microtablet, a pellet, a micropellet, a capsule, a capsule containing microtablets or a liquid formulation.
